# EUROPÄISCHE PATENTSCHRIFT

(11) **EP 0 727 453 B1**
(45) Veröffentlichungstag und Bekanntmachung des Hinweises auf die Patenterteilung: **20.05.1998**
(21) Anmeldenummer: 96101594.8
(22) Anmeldetag: 05.02.1996
(51) Int. Cl.: C08G 18/79, C07D 251/34

(54) **Isocyanurat-Polyisocyanate auf Basis von 1,3-Diisocyanatocycloalkanen**
Isocyanurate polyisocyanates made from 1,3-diisocyanatecycloalcanes
Isocyanurate polyisocyanate à base de 1,3-diisocyanatecycloalcanes

(30) Priorität: 17.02.1995 DE 19505351
(43) Veröffentlichungstag der Anmeldung: 21.08.1996
(73) Patentinhaber: BAYER AG, 51368 Leverkusen (DE)
(72) Erfinder: Richter, Frank, Dr., D-51373 Leverkusen (DE); Mertes, Harald, Dr., D-50670 Köln (DE); Halpaap, Reinhard, Dr., D-51519 Odenthal (DE); Pedain, Josef, Dr., D-51061 Köln (DE); Becker, Gernot, Dr., D-41540 Dormagen (DE)

(56) Entgegenhaltungen:
- EP-A- 0 017 998
- EP-A- 0 668 271
- DE-A- 4 412 327
- DATABASE WPI Week 4387 Derwent Publications Ltd., London, GB; AN 87-304355 XP002004400 & JP-A-62 215 619 (DAINIPPON INK CHEM KK)

## Beschreibung

Die Erfindung betrifft ein neues Verfahren zur Herstellung von Isocyanuratgruppen aufweisenden Polyisocyanaten durch katalytische Trimerisierung eines Teils der Isocyanatgruppen von (cyclo)aliphatischen Diisocyanaten, die ausschließlich oder überwiegend aus 1,3-Diisocyanatocycloalkanen mit trans-ständigen Isocyanatgruppen bestehen, die so erhältlichen, Isocyanuratgruppen aufweisenden Polyisocyanate und ihre Verwendung als Isocyantkomponente in Polyurethanlacken.

Isocyanuratgruppen aufweisende Polyisocyanate sind seit langem bekannt. Ihre Herstellung erfolgt im allgemeinen durch katalytische Trimerisierung eines Teils der Isocyanatgruppen von organischen Diisocyanaten, Abbruch der Trimerisierungsreaktion beim gewünschten Trimerisierungsgrad und anschließende Entfernung von nicht umgesetztem Ausgangsdiisocyanat. Einen sehr guten Überblick über die zu diesem Thema vorliegende Literatur findet man in J. prakt. Chem. 336 (1994) 185 ff.

Die bekannten Verfahren eignen sich jedoch nicht zur Herstellung von Isocyanuratgruppen aufweisenden Polyisocyanaten aus 1,3-Diisocyanatocycloalkanen wie z.B. dem Hexahydro-2,4- bzw. -2,6-diisocyanatotoluol (H₆TDI). Bei Verwendung derartiger Ausgangsdiisocyanate erhält man anstelle der angestrebten Trimerisate Produkte, die nach Entfernung des überschüssigen Monomeren erheblich zu niedrige NCO-Gehalte und untypisch hohe Schmelzpunkte und -viskositäten aufweisen. Die Anwesenheit der erwarteten Isocyanurat-Strukturelemente kann aufgrund analytischer Untersuchungen (IR, ¹³C-NMR, GPC) an den so erhaltenen Produktgemischen (vgl. Beispiel 1) mit Sicherheit ausgeschlossen werden.

Andere Diisocyanate mit cycloaliphatisch gebundenen Isocyanatgruppen wie beispielsweise 3-(Isocyanatomethyl)-3,5,5-trimethylcyclohexylisocyanat (Isophorondiisocyanat, IPDI) bzw. 4,4'-Diisocyanatodicyclohexylmethan (H₁₂MDI) zeigen diese Anomalie nicht und lassen sich problemlos zu den entsprechenden Isocyanuratgruppen aufweisenden Polyisocyanaten trimerisieren, wie z.B. in EP-A- 0 003 765 bzw. EP-A 0 017 998 beschrieben wird.

Ursache für die beobachteten Probleme bei Versuchen zur Trimerisierung von 1,3-Diisocyanatocycloalkanen ist die Bildung cyclopolymerer Verbindungen vom α-Nylon-Typ, deren Synthese - allerdings unter anderen Reaktionsbedingungen - vorbeschrieben ist. So fanden Corfield und Crawshaw (Chem. Commun. **4** (1966), 85), daß cis-1,3-Diisocyanatocyclohexan in Dimethylformamidlösung bei -60°C unter Natriumcyanidkatalyse umgesetzt einen weißen Feststoff lieferte, der nach IR-Analyse keine freien NCO-Gruppen enthielt und dem die Struktur eines Cyclopolymerisates **1a** bzw. **1b** zugeordnet wurde. Naegele und Wendisch (Organic Magnetic Resonance **2** (1970) 439) erwähnten die starke Verharzungstendenz des cis,cis-1-Methyl-2,4-diisocyanatocyclohexans, ohne jedoch Beweise für die postulierte α-Nylon-Struktur der resultierenden Harze anzuführen. Die cis-und trans-Formen der isomeren 1,3,5-Triisocyanatocyclohexane, in denen jeweils mindestens zwei Isocyanatgruppen cis-ständig zueinander angeordnet sind, können ebenfalls leicht cyclopolymerisiert werden (J. Makromol. Sci.-Chem. **A5** (1)(1971) 37). Die EP 0 014 365 schließlich beschreibt die Cyclopolymerisation der cis-Anteile des technisch anfallenden H₆TDI-Isomerengemisches, katalysiert durch Blei-II-salze, tertiäre Amine, Alkalimetall-hydroxide oder auch Alkalimetallalkoholate, bei höheren Temperaturen.

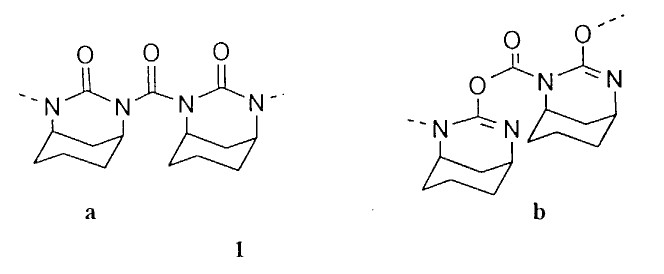

In keiner der genannten Veröffentlichungen finden sich jedoch Hinweise auf die Bildung von Polyisocyanaten mit Isocyanuratstruktur, obwohl diese, z.B. von Butler und Corfield bei der Modifizierung der isomeren 1,3,5-Triisocyanatocyclohexane, beschrieben in J. Makromol. Sci.-Chem. **A5** (1) (1971) 37, ausdrücklich angestrebt wurde. Zwar werden 1,3-Diisocyanatocycloalkane in zahlreichen Patenten, die die Trimerisierungsreaktionen von Diisocyanaten beschreiben, zusammen mit vielen anderen Diisocyanaten aufgelistet, konkrete Beispiele einer erfolgreichen Trimerisierung der 1,3-Diisocyanatocycloalkane werden jedoch nicht angeführt. So berichtete Iwakura in J. Polym. Sci, Part A-1. 6, 261 (1968) von erfolglosen Versuchen durch katalytische Trimerisierung von Cyclohexylisocyanat zu dem entsprechenden 1,3,5-Tricyclohexylisocyanurat zu gelangen.

1,3-Diisocyanatocycloalkane sind erst in neuerer Zeit durch Gasphasenphosgenierung der entsprechenden cycloaliphatischen Diamine technisch leicht zugänglich geworden (Deutsche Patentanmeldung P 44 12 327.2) und stellen besonders interessante Ausgangsmaterialien zur Herstellung von lichtechten, Isocyanuratgruppen aufweisenden Lackpolyisocyanaten dar. Solche Lackpolyisocyanate würden ausschließlich sekundär gebundene NCO-Gruppen und damit eine einheitliche Reaktivität aufweisen und im übrigen vor allem auf vergleichsweise niedermolekularen Diisocyanaten basieren, die im Unterschied zu Diisocyanatodicyclohexylmethanen im Anschluß an die Trimerisierungsreaktion problemlos durch Dünnschichtdestillation aus den Trimerisaten eliminiert werden können.

Es war daher die der Erfindung zugrundeliegende Aufgabe, unter Umgehung der oben skizzierten Schwierigkeiten einen Weg zu derartigen Isocyanuratgruppen aufweisenden Polyisocyanaten zu eröffnen.

Diese Aufgabe konnte mit dem nachstehend näher beschriebenen erfindungsgemäßen Verfahren gelöst werden. Diesem Verfahren liegt die Beobachtung zugrunde, daß auch 1,3-Diisocyanatocycloalkane problemlos trimerisiert werden können, wenn sie vorab von den Isomeren mit cis-ständigen Isocyanatgruppen bis auf einen Restgehalt von maximal 20 Gew.-% befreit werden. Beim nachstehend näher beschriebenen erfindungsgemäßen Verfahren werden demzufolge als Ausgangsdiisocyanate solche eingesetzt, die pro 100 Gew.-Teilen an 1,3-Diisocyanatocycloalkanen mit trans-ständigen Isocyanatgruppen max. 25 Gew.-Teile an entsprechenden Diisocyanaten mit cis-ständigen Isocyanatgruppen aufweisen.

Gegenstand der Erfindung ist ein Verfahren zur Herstellung von Isocyanuratgruppen aufweisenden Polyisocyanaten mit einem NCO-Gehalt von 10 bis 30 Gew.-% durch katalytische Trimerisierung eines Teils der Isocyanatgruppen von (cyclo)aliphatischen Diisocyanaten, Abbruch der Trimerisierungsreaktion nach Erreichen des angestrebten Trimerisierungsgrades und Entfernung von überschüssigem, nicht umgesetztem Ausgangsdiisocyanat, dadurch gekennzeichnet, daß man als Ausgangsdiisocyanate (cyclo)aliphatische Diisocyanate oder Gemische von (cyclo)aliphatischen Diisocyanaten, bestehend aus
(i) 100 Gew.-Teilen an 1,3-Diisocyanatocycloalkanen des Molekulargewichtsbereichs 138 bis 300 mit trans-ständigen Isocyanatgruppen,
(ii) 0 bis 25, vorzugsweise 0 bis 10 Gew.-Teilen an 1,3-Diisocyanatocycloalkanen des Molekulargewichtsbereichs 138 bis 300 mit cis-ständigen Isocyanatgruppen
und
(iii) 0 bis 300 vorzugsweise 0 bis 100 Gew.-Teilen an anderen (cyclo)aliphatischen Diisocyanaten des Molekulargewichtsbereichs 168 bis 300
verwendet.

Gegenstand der Erfindung sind auch die nach diesem Verfahren erhältlichen, Isocyanuratgruppen aufweisenden Polyisocyanate.

Gegenstand der Erfindung ist auch die Verwendung der erfindungsgemäßen Isocyanuratgruppen aufweisenden Polyisocyanate, gegebenenfalls in mit Blockierungsmitteln für Isocyanuratgruppen blockierter Form, als Bindemittel oder Bindemittelkomponente in 1 K- oder 2 K-Lacken.

Beispiele für erfindungsgemäß einzusetzende 1,3-Diisocyanatocycloalkane sind solche des Molekulargewichtsbereichs 138 bis 300, vorzugsweise 180 bis 250. Der Begriff "Diisocyanatocycloalkan" soll bedeuten, daß beide Isocyanatgruppen direkt mit einem cycloaliphatischen Ring verknüpft sind. Dieser cycloaliphatische Ring weist im allgemeinen 4 bis 8, vorzugsweise 5 oder 6 und besonders bevorzugt 6 Kohlenstoffatome auf und kann beliebige inerte Substituenten, insbesondere Alkylsubstituenten, vorzugsweise solche mit 1 bis 3 Kohlenstoffatomen aufweisen. Besonders bevorzugt sind solche 1,3-Diisocyanatocyclohexane, die in 2- und/oder in 4-Position einen C₁-C₃-Alkylsubstituenten aufweisen. Geeignete 1,3-Diisocyanatocyclohexane sind beispielsweise 1,3-Diisocyanatocyclobutan 1,3-Diisocyanatocyclopentan, 1,3-Diisocyanatocyclohexan, 1,3-Diisocyanatocycloheptan, 1,3-Diisocyanatocycloheptan, 1,3-Diisocyanato-2-methylcyclohexan, 1,3-Diisocyanato-4-methylcyclohexan, sowie beliebige technische Gemische der beiden letztengenannten Diisocyanate, 1,3-Diisocyanato-2-isopropylcyclohexan, 1,3-Diisocyanato-2-methylcyclohexan, 1,3-Diisocyanato-2,4-dimethylcyclohexan, 1,3-Diisocyanato-2,4-diethylcyclohexan, 1,3-Diisocyanato-2,4-diethyl-6-methylcyclohexan, 1,3-Diisocyanate-2-methyl-4,6-diethylcyclohexan, sowie beliebige Gemische der beiden letztengenannten Diisocynate oder 1,3-Diisocyanato-2,4,6-triisobutyl-cyclohexan.

Bei diesen 1,3-Diisocyanatocycloalkanen handelt es sich um literaturbekannte Verbindungen, die auf besonders einfache Weise durch Gasphasenphosgenierung der ihnen zugrundeliegenden Diamine hergestellt werden können. Diese Gasphasenphosgenierung ist in der Deutschen Patentanmeldung P 44 12 327.2 beschrieben.

Die durch Phosgenierung der entsprechenden Diamine erhaltenen Diisocyanate stellen cis-trans-Isomerengemische mit einem cis-trans-Gewichtsverhältnis von 5:1 bis 1:1, vorzugsweise 3:1 bis 2:1, dar und sind als solche noch nicht als Ausgangsmaterial für das erfindungsgemäße Verfahren geeignet. Voraussetzung hierfür ist eine Entfernung zumindest der Hauptmenge der cis-Isomeren, so daß Diisocyanate bzw. Diisocyanatgemische resultieren, deren cis-trans-Isomerenverhältnis den oben gemachten Ausführungen entspricht.

Zur Abtrennung der cis-Isomeren eignet sich deren Überführung in kristalline Cyclopolymerisate vom α-Nylon-Typ nach der Lehre der EP-A-0 014 365. Hierzu kann beispielsweise das Ausgangsgemisch mit 50 ppm (Gewicht) bis 1 Gew.-%, bezogen auf Ausgangsgemisch, eines geeigneten Polymerisationskatalysators versetzt und unter selektiver Polymerisation der cis-Isomeren auf 0 bis 250°C erhitzt werden. Anschließend kann das Reaktionsgemisch in einem Lösungsmittel, welches auch die Polymerisate löst, aufgenommen werden, worauf sich die Ausfällung durch Vermischen der Lösung mit einem Fällungsmittel anschließt.

Geeignete Polymerisationskatalysatoren sind beispielsweise Blei-II-acetat, tertiäre Amine wie Triethylendiamin, N,N-Dimethylaminoethanol, N,N,N',N'-Tetramethylethylendiamin, Alkalimetallhydroxide oder -alkoholate wie Natriumhydroxid, Kaliumhydroxid, Natrium-ethylat oder Kalium-ethylat oder Ammoniumbasen wie Trimethylbenzylammoniumhydroxid (®Triton B, Hersteller: Merck AG, Germany), Methylcholin, Tris-(dimethylaminopropyl)-hexahydrotriazin (®Polycat 41, Hersteller: Air Products) oder Hexamethyldisilazan (HMDS). Besonders überraschend ist hierbei, daß die letztgenannten Ammoniumbasen ausgezeichnete Trimerisierungskatalysatoren für organische Isocyanate sind und dennoch unter den genannten Reaktionsbedingungen selektive Polymerisationskatalysatoren für die cis-Isomeren darstellen und erst nach deren Entfernung als Trimerisierungskatalysator wirken.

Geeignete Lösungsmittel sind beispielsweise Xylol, Toluol oder höhere Alkylbenzole bzw. deren Gemische.

Geeignete Fällungsmittel sind beispielsweise aliphatische Kohlenwasserstoffe wie n-Hexan.

Nach der selektiven Ausfällung der Cyclopolymerisate vom α-Nylon-Typ und deren Eliminierung durch Filtration können die hierbei resultierenden Filtrate als solche für das erfindungsgemäße Verfahren eingesetzt werden. Vorzugsweise werden jedoch die Filtrate durch Destillation vom Lösungsmittel/Fällungsmittel-Gemisch befreit und die resultierenden Destillationsrückstände gegebenenfalls nach destillativer Reindarstellung für das erfindungsgemäße Verfahren verwendet.

Vor der Durchführung des erfindungsgemäßen Verfahrens können die auf diese Weise erhaltenen, an trans-Isomeren angereicherten Diisocyanate mit anderen (cyclo)aliphatischen Diisocyanaten, die nicht der oben gemachten Definition bezüglich der erfindungswesentlichen Diisocyanate entsprechen, abgemischt werden, so daß als Ausgangsmaterialien für das erfindungsgemäße Verfahren Diisocyanate bzw. -Diisocyanatgemische der vorstehend genannten Zusammensetzung resultieren.

Geeignete (cyclo)aliphatische Diisocyanate, die gegebenenfalls mit den erfindungswesentlichen Ausgangsmaterialien vor der Trimerisierungsreaktion abgemischt werden, sind beispielsweise Hexamethylendiisocyanat (HDI), 1-Isocyanato-3,3,5-trimethyl-5-isocyanatomethylcyclohexan (IPDI) oder 4,4'-Diisocynatodicyclohexylmethan (H₁₂ MDI).

Die Trimerisierungsreaktion erfolgt nach an sich bekannten Methoden wie sie beispielsweise in EP-A-0 003 765 beschrieben sind. Geeignete Trimerisierungskatalysatoren sind solche der an sich bekannten Art, gut geeignet ist beispielsweise eine 0,5 bis 10 gew.-%ige Lösung von ®Triton B in einem alkoholischen Lösungsmittel wie beispielsweise Methanol und/oder 2-Ethylhexan-1,3-diol.

Zur Durchführung der Trimerisierungsreaktion kann beispielsweise das als Ausgangsmaterial eingesetzte Diisocyanat bzw. Diisocyanatgemisch mit der genannten Katalysatorlösung versetzt werden, wobei der Katalysator, bezogen auf gelöstes Triton B, in einer Menge von 50 bis 500, vorzugsweise 100 bis 300 ppm (Gewicht) zum Einsatz gelangt. Die Umsetzung erfolgt bei 20 bis 180, vorzugsweise 40 bis 80°C, unter Einhaltung eines Trimerisierungsgrades von 5 bis 30, vorzugsweise 10 bis 25 %. Der Trimerisierungsgrad gibt hierbei den Prozentsatz der im Ausgangsgemisch vorliegenden Isocyanatgruppen an, der während der Trimerisierungsreaktion abreagiert. Im allgemeinen wird ohne Lösungsmittel, abgesehen von dem mit dem Katalysator eingebrachten Lösungsmittel, gearbeitet. Die Abstoppung der Umsetzung erfolgt durch thermische Desaktivierung des Katalysators oder durch Zugabe eines geeigneten Katalysatorgifts. Im Falle der Verwendung von Triton B als Katalysator ist beispielsweise Dibutylphosphat als Katalysatorgift geeignet.

Im Anschluß an die Trimerisierungsreaktion wird im allgemeinen monomeres Ausgangsdiisocyanat durch Destillation im Dünnschichtverdampfer bis auf einen Restgehalt von max. 0,5 Gew.-% entfernt. Die hierbei als Destillationsrückstand anfallenden erfindungsgemäßen Verfahrensprodukte stellen farblose bis schwach gelb gefärbte, im Temperaturbereich von 40 bis 150°C schmelzende Festharze mit einem NCO-Gehalt von 10 bis 30, vorzugsweise 13 bis 25 Gew.-% dar.

Die erfindungsgemäßen Verfahrensprodukte eignen sich als Bindemittel oder Bindemittelkomponente in Beschichtungsmaterialien. Vorzugsweise werden sie als Vernetzerkomponente in 2-K-Beschichtungsmaterialien eingesetzt, sie können jedoch auch als alleinige Bindemittel in, unter dem Einfluß von Luftfeuchtigkeit aushärtenden 1-K-Systemen, eingesetzt werden. In mit Blockierungsmitteln für Isocyanatgruppen blockierter Form eignen sie sich im übrigen als Härter für Einbrennlacke. Reaktionspartner für die erfindungsgemäßen Verfahrensprodukte in den genannten 2-K-Beschichtungsmittel oder auch in den genannten Einbrennlacken sind die an sich bekannten hydroxyfunktionellen Polyester, Polyether, Polycarabonate, Polyurethane oder Polyacrylate.

Beschichtungsmittel, die als wesentliches Bindemittel bzw. als Härter gegebenenfalls blockierte erfindungsgemäße Verfahrensprodukte enthalten, können neben den beispielhaft genannten Polyhydroxylverbindungen selbstverständlich die üblichen Hilfs- und Zusatzmittel enthalten. Beispielhaft genannt seien Lösungsmittel wie Butylacetat, Ethylacetat, Methoxypropylacetat, Ethylglykolacetat, Methylethylketon, Methylisobutylketon, Toluol, Xylol, Testbenzine und Gemische derartiger Lösungsmittel. Weitere Hilfs- und Zusatzmittel sind z.B. Benetzungsmittel, Verlaufsmittel, Hautverhinderungsmittel, Antischaummittel, Mattierungsmittel, viskositätsregulierende Stoffe, Pigmente, Farbstoffe, UV-Absorber und/oder Stabilisatoren gegen thermische und oxidative Einflüsse.

Die Beschichtungsmittel auf Basis der erfindungsgemäßen Verfahrensprodukte können zur Beschichtung einer Vielzahl von Materialien dienen, wie z.B. Holz, Kunststoffe, Leder, Metalle, Papier, Beton, Mauerwerk, Keramik und Textilien.

Die nachfolgenden Beispiele und Vergleichsbeispiele sollen die Erfindung näher erläutern, ohne sie jedoch einzuschränken. Alle Prozentangaben beziehen sich auf das Gewicht.

### Beispiele

### Beispiel 1

(Vergleichsbeispiel unter Verwendung eines Gemischs aus 80 % 1,3-Diisocyanato-4-methylcyclohexan und 20 % 1,3-Diisocyanato-2-methylcyclohexan mit einem cis-trans-Gewichtsverhältnis von 2:1 "H₆TDI")

450 g H₆TDI werden in einem Dreihalskolben mit Stickstoffeinleitungsrohr, das bis in die Flüssigkeit ragt, Innenthermometer, Rückflußkühler, Dosiereinrichtung für die Katalysatorlösung und mechanischem Rührer eine Stunde bei Zimmertemperatur im Vakuum (< 0.1 mbar) entgast, anschließend mit trockenem Stickstoff belüftet und unter Durchleiten von Stickstoff eine Stunde bei 60°C gerührt. Anschließend werden bei einer Innentemperatur von 65°C langsam 3,6 g einer 0,5 %igen Triton® B Lösung (entprechend 40 ppm Triton® B) in einem Gemisch von 2-Ethylhexan-1,3-diol und Methanol im Mischungsverhältnis 130:1 zugegeben, wobei unter exothermer Reaktion, die durch Entfernen der Außenheizung und ggf. externe Kühlung unter 80°C gehalten wird, und Anstieg der Viskosität der Reaktionsmischung ein titrimetrisch bestimmter NCO-Gehalt von 39 % (U_{NCO} = 16,3 %) erreicht wird. Anschließend wird durch Zugabe von 0,01 g Dibutylphosphat abgestoppt.

Die so erhaltene Reaktionsmischung läßt sich nicht durch Dünnschichtdestillation in einem Kurzwegverdampfer aufarbeiten. Unmittelbar an der Eintropfstelle bilden sich hochviskose Ablagerungen, die selbst bei 200°C nicht schmelzen.

Eine detaillierte analytische Untersuchung des erhaltenen Reaktionsgemisches belegt, daß neben kleinen Mengen niedermolekularer Verbindungen, die jedoch keine Isocyanuratgruppen aufweisen, im wesentlichen cyclopolymeres α-Nylon entstanden ist.

Zu völlig analogen Ergebnissen gelangt man,.wenn anstelle von H₆TDI 1,3-Diisocyanato-4-isopropylcydohexan (H₆CDI) verwendet wird (vgl. Beispiel 4).
Der Umsetzungsgrad hat auf den Reaktionsverlauf keinen Einfluß; wird die oben beschriebene Umsetzung ohne Unterbrechung der Reaktion bis zum Erreichen eines konstanten NCO-Wertes durchgeführt, liegt nahezu der gesamte cis-Isomerenanteil in Form von α-Nylon vor, keines der Nebenprodukte weist eine Isocyanuratstruktur auf und die Reaktion kommt nach der vollständigen Umsetzung der cis-Anteile selbständig zum Stillstand.

### Beispiel 2 (erfindungsgemäß)

100 g H₆TDI der o.g. Isomerenzusammensetzung und 2 g Blei-II-acetat werden in einer Dreihalskolbenrührapparatur vorgelegt und unter Rühren auf 125°C erhitzt. Nach ca. 30 min hat die exotherme Reaktion begonnen. Wenn die Innentemperatur 150°C überschreitet oder die Viskosität der Mischung zu stark ansteigt, um gute Rührbarkeit zu gewährleisten, werden 50 -100 ml Xylol (Isomerengemisch) zugesetzt und man läßt unter Rühren abkühlen. Anschließend wird die Mischung unter kräftigem Rühren in 500 ml n-Hexan getropft wobei sich die α-Nylonverbindung als farbloser bis hellgelber Feststoff absetzt. Nach Filtration und Waschen des Niederschlags werden die Filtrate vom Lösungsmittel befreit und im Vakuum destilliert, das Gemisch der trans-Isomeren geht bei einem Druck von 5 mbar bei 105-106°C über, 30-32 g Ausbeute = 90-95 % d.Th. Der Gehalt des Gemischs an cis-Isomeren liegt unter 2 %.

313,6 g des so erhaltenen trans-Isomerengemisches werden in einem Dreihalskolben mit Stickstoffeinleitungsrohr, das bis in die Flüssigkeit ragt, Innenthermometer, Rückflußkühler, Dosiereinrichtung für die Katalysatorlösung und mechanischem Rührer eine Stunde bei Zimmertemperatur im Vakuum (< 0.1 mbar) entgast, anschließend mit trockenem Stickstoff belüftet und unter Durchleiten von Stickstoff eine Stunde bei 60°C gerührt. Anschließend wird bei einer Innentemperatur von 65°C über einen Zeitraum von ca. 90 min eine 0,5 %ige Triton B Lösung zugegeben, wobei unter leicht exothermer Reaktion (Innentemperatur 65°C) ein titrimetrisch bestimmter NCO-Gehalt von 43,7 % erreicht wird, der nach einstündigem Nachrühren bei 70°C weiter auf 41,9 % abfällt (U_{NCO} = 10 %). Anschließend wird durch Zugabe von 0,06 g Dibutylphosphat abgestoppt. Die leicht gelbliche, klare Lösung wird bei 170°C und 0,2 mbar gedünnschichtet, wobei 42 g eines Festharzes mit einem NCO-Gehalt von 19,6 % und einem Schmelzbereich von ca. 80-100°C erhalten werden. Das Trimerisat ist in Methylenchlorid, Chloroform, Methoxypropylacetat/Xylol-Gemisch (1:1) und ®Solvesso 100 gut löslich. Die Lösungen zeigen eine gute Verträglichkeit mit üblichen Lackpolyolen.

### Beispiel 3 (erfindungsgemäß)

261,8 g "trans-H₆TDI", hergestellt gemäß Beispiel 2, werden wie in Beispiel 2 beschrieben mit 0,683 g einer 10 %igen Triton® B Lösung bei 60-70°C bis zu einem Trimerisierungsgrad von 20 % trimerisiert, anschließend mit 0,1 g 25 %iger Dibutylphosphatlösung abgestoppt und durch Dünnschichtdestillation wie unter 2 beschrieben aufgearbeitet. Man erhält ein Festharz mit einem NCO-Gehalt von 19,3 %.

### Beispiel 4 (erfindungsgemäß)

100 g eines 1,3-Diisocyanato-4-isopropylcyclohexan-Isomerengemisches (H₆CDI) mit einem cis-Isomerenanteil von ca. 50 % werden in einer Apparatur wie in Beispiel 2 beschrieben bei einer Innentemperatur der Mischung von ca. 60°C mit 0,26 g einer 10 %igen Triton® B Lösung tropfenweise versetzt wobei ohne nennenswerten Temperaturanstieg ein deutliches Ansteigen der Viskosität beobachtet wird. Nach Zugabe von 30 g Xylol wird noch zwei Stunden bei 60°C nachgerührt, anschließend in 1 l n-Hexan gegeben und nach Abtrennung vom unlöslichen Polymer destillativ aufgearbeitet. Man erhält 48g = 96 % d. Th. trans-H₆CDI mit einem NCO-Gehalt von 40,1 % (theor. 40,3 %) und einem Siedebereich von 58-61°C bei einem Druck von 0,05 mbar. Der cis-Gehalt liegt unter 2 %.

165,6 g des so erhaltenen trans-H₆CDI werden in der im Beispiel 3 beschriebenen Weise mit 0,41 g 10 %iger Triton® B Lösung bis zu einem Trimersierungsgrad von 21 % trimerisiert und anschließend mit 0,08 g Dibutylphosphat abgestoppt. Die klare Lösung wird dünnschichtdestillativ aufgearbeitet wobei ein Festharz mit einem NCO-Gehalt von 15,1 % erhalten wird.

IR- und NMR-spektroskopische Untersuchungen und die Resultate der Gelpermeationschromatographie belegen, daß es sich bei den in den Beispielen 2-4 beschriebenen Produkten im wesentlichen um Gemische oligomerer Isocyanurate handelt.

## Patentansprüche

1. Verfahren zur Herstellung von Isocyanuratgruppen aufweisenden Polyisocyanaten mit einem NCO-Gehalt von 10 bis 30 Gew.-% durch katalytische Trimerisierung eines Teils der Isocyanatgruppen von (cyclo)aliphatischen Diisocyanaten, Abbruch der Trimerisierungsreaktion nach Erreichen des angestrebten Trimerisierungsgrades und Entfernung von überschüssigem, nicht umgesetztem Ausgangsdiisocyanat, dadurch gekennzeichnet, daß man als Ausgangsdiisocyanate (cyclo)aliphatische Diisocyanate oder Gemische von (cyclo)aliphatischen Diisocyanaten, bestehend aus
(i) 100 Gew.-Teilen an 1,3-Diisocyanatocycloalkanen des Molekulargewichtsbereichs 138 bis 300 mit trans-ständigen Isocyanatgruppen,
(ii) 0 bis 25 Gew.-Teilen an 1,3-Diisocyanatocycloalkanen des Molekulargewichtsbereichs 138 bis 300 mit cis-ständigen Isocyanatgruppen
und
(iii) 0 bis 300 Gew.-Teilen an anderen (cyclo)aliphatischen Diisocyanaten des Molekulargewichtsbereichs 168 bis 300
verwendet.

2. Verfahren gemäß Anspruch 1, dadurch gekennzeichnet, daß man als Diisocyanate (i) und gegebenenfalls (ii) 1,3-Diisocyanatcyclohexane mit C₁-C₃-Alkylsubstituenten in 2- und/oder 4-Position verwendet.

3. Verfahren gemäß Anspruch 1 und 2, dadurch gekennzeichnet, daß man als Diisocyanate (i) und gegebenenfalls (ii) 1,3-Diisocyanato-2-methylcyclohexan, 1,3-Diisocyanato-4-methylcyclohexan, 1,3-Diisocyanato-2-isopropylcyclohexan, 1,3-Diisocyanato-4-isopropylcyclohexan oder beliebige Gemische dieser Diisocyanate verwendet.

4. Verfahren gemäß Anspruch 1 bis 3, dadurch gekennzeichnet, daß die bei der Trimerisierungsreaktion einzusetzenden Diisocyanate aus
100 Gew.-Teilen 1,3-Diisocyanatocycloalkanen mit trans-ständigen Isocyanatgruppen und
0 bis 10 Gew.-Teilen 1,3-Diisocyanatocycloalkanen mit cis-ständigen Isocyanatgruppen
bestehen.

5. Gemäß Anspruch 1 bis 4 erhältliche, Isocyanuratgruppen aufweisende Polyisocyante.

6. Verwendung der gemäß Anspruch 1 bis 4 erhältlichen, Isocyanuratgruppen aufweisenden Polyisocyanate, gegebenenfalls in mit Blockierungsmitteln für Isocyanatgruppen blockierter Form, als Bindemittel oder Bindemittelkomponente in 1-K- oder 2-K-Lacken.

## Claims

1. A method of producing polyisocyanates which contain isocyanurate groups and which have an NCO content of 10 to 30 % by weight by the catalytic trimerisation of part of the isocyanate groups of (cyclo)aliphatic diisocyanates, terminating the trimerisation reaction after the sought-after degree of trimerisation has been reached and removing excess, unreacted diisocyanate starting material, characterised in that (cyclo)aliphatic diisocyanates or mixtures of (cyclo)aliphatic diisocyanates, comprising
(i) 100 parts by weight of 1,3-diisocyanatocycloalkanes of molecular weight range 138 to 300, having isocyanate groups in the trans position,
(ii) 0 to 25 parts by weight of 1,3-diisocyanatocycloalkanes of molecular weight range 138 to 300, having isocyanate groups in the cis position,
and
(iii) 0 to 300 parts by weight of other (cyclo)aliphatic diisocyanates of molecular weight range 168 to 300
are used as the diisocyanate starting materials.

2. A method according to claim 1, characterised in that 1,3-diisocyanatocyclohexanes with C₁-C₃ alkyl substituents in the 2- and/or 4-position are used as diisocyanates (i) and optionally (ii).

3. A method according to claims 1 or 2, characterised in that 1,3-diisocyanato-2-methylcyclohexane, 1,3-diisocyanato-4-methylcyclohexane, 1,3-diisocyanato-2-isopropylcyclohexane, 1,3-diisocyanato-4-isopropylcyclohexane or any mixtures of these diisocyanates are used as diisocyanates (i) and optionally (ii).

4. A method according to claims 1 to 3, characterised in that the diisocyanates used in the trimerisation reaction comprise
100 parts by weight of 1,3-diisocyanatocycloalkanes having isocyanate groups in the trans position, and
0 to 10 parts by weight of 1,3-diisocyanatocycloalkanes having isocyanate groups in the cis position.

5. Polyisocyanates which contain isocyanurate groups and which are obtainable according to claims 1 to 4.

6. The use of the polyisocyanates which contain isocyanurate groups and which are obtainable according to claims 1 to 4, optionally in a form which is blocked with blocking agents for isocyanate groups, as a binder vehicle or binder vehicle component in single-component or two-component lacquers.

## Revendications

1. Procédé de préparation de polyisocyanates comportant des groupes isocyanurate ayant une teneur en NCO de 10 à 30% en masse par trimérisation catalytique d'une partie des groupes isocyanate de diisocyanates (cyclo)aliphatiques, interruption de la réaction de trimérisation après obtention du degré de trimérisation recherché et élimination du diisocyanate initial qui n'a pas réagi en excès, caractérisé en ce que l'on utilise comme diisocyanates initiaux des diisocyanates (cyclo)aliphatiques ou des mélanges de diisocyanates (cyclo)aliphatiques consistant en :
(i) 100 parties en masse de 1,3-diisocyanatocycloalcanes d'un domaine de masse moléculaire de 138 à 300 groupes isocyanate en position trans,
(ii) 0 à 25 parties en masse de 1,3-diisocyanatocycloalcanes d'un domaine de masse moléculaire de 138 à 300 à groupes isocyanate en position cis,
et
(iii) 0 à 300 parties en masse d'autres diisocyanates (cyclo)aliphatiques d'un domaine de masse moléculaire de 168 à 300.

2. Procédé selon la revendication 1, caractérisé en ce que l'on utilise comme diisocyanates (i) et éventuellement (ii) des 1,3-diisocyanatocyclohexanes ayant des substituants alkyle en C₁-C₃ en positions 2 et/ou 4.

3. Procédé selon les revendications 1 et 2, caractérisé en ce que l'on utilise comme diisocyanates (i) et éventuellement (ii) le 1,3-diisocyanato-2-méthylcyclohexane, le 1,3-diisocyanato-4-méthylcyclohexane, le 1,3-diisocyanato-2-isopropyl-cyclohexane, le 1,3-diisocyanato-4-isopropylcyclohexane ou des mélanges quelconques de ces diisocyanates.

4. Procédé selon les revendications 1 à 3, caractérisé en ce que les diisocyanates à utiliser lors de la réaction de trimérisation consistent en :
100 parties en masse de 1,3-diisocyanatocycloalcanes à groupes isocyanate en position trans et
0 à 10 parties en masse de 1,3-diisocyanatocycloalcanes à groupes isocyanate en position cis.

5. Polyisocyanates comportant des groupes isocyanurate qui peuvent être obtenus selon les revendications 1 à 4.

6. Utilisation des polyisocyanates comportant des groupes isocyanurates qui peuvent être obtenus selon les revendications 1 à 4, éventuellement sous forme bloquée avec des agents de blocage pour groupes isocyanate, comme liant ou composant de liant dans des peintures à un composant ou à deux composants.
